# EUROPEAN PATENT APPLICATION

(11) **EP 3 730 049 A1**
(43) Date of publication of application: **28.10.2020**
(21) Application number: 20165867.1
(22) Date of filing: 26.03.2020
(51) Int. Cl.: A61B 5/0452, A61B 5/0468, A61B 5/0472, A61B 5/00

(54) **APPARATUS AND METHOD FOR ANALYZING ELECTROCARDIOGRAM**

(30) Priority: 26.04.2019 KR 20190049278
(71) Applicant: Samsung SDS Co., Ltd., Seoul 05510 (KR)
(72) Inventor: JUNG, Eunsoo, 05510 Seoul (KR); YUN, Yongkeun, 05510 Seoul (KR); SON, Hyeonggwan, 05510 Seoul (KR); OH, Kyusam, 05510 Seoul (KR); JEON, Eunjoo, 05510 Seoul (KR); KWON, Soonhwan, 05510 Seoul (KR)
(74) Representative: Grünecker Patent- und Rechtsanwälte PartG mbB

(57) **Abstract**

Disclosed are an apparatus and method for analyzing electrocardiogram. The electrocardiogram analysis apparatus includes an inputter configured to receive an electrocardiogram of a subject, a beat analyzer configured to first detect and classify one or more normal beats among a plurality of beats included in the received electrocardiogram, request beat classification for remaining beats except for the one or more normal beats from a remote diagnosis server, and label each of the plurality of beats according to a normal beat detection result and a beat classification result received from the remote diagnosis server, and a rhythm analyzer configured to perform rhythm analysis on the electrocardiogram based on a labeling result.

## Description

### CROSS-REFERENCE TO RELATED APPLICATION

This application claims the benefit of Korean Patent Application No. 10-2019-0049278, filed on April 26, 2019, the disclosures of which are incorporated herein by reference for all purposes.

### BACKGROUND

### 1. Field

Embodiments of the present disclosure relate to a technique for analyzing electrocardiograms.

### 2. Discussion of Related Art

An electrocardiogram is a recording of, by analyzing heart's electrical activity, the electrical activity in wavelength form. Electrocardiogram analysis is essentially used for diagnosing heart disease, in particular, for diagnosing cardiac arrhythmia with an irregular heartbeat. The electrocardiogram analysis is utilized for diagnosing myocardial disorders, enlargement of atrial ventricles, dilatation, pulmonary circulation disorders, electrolyte metabolic abnormalities, drug effects, other heart diseases, and related diseases, in addition to the diagnosis of arrhythmia.

Since an arrhythmia signal does not always appear in an electrocardiogram, it is necessary to observe a long-term (e.g., for 24 hours) measurement signal for diagnosis of arrhythmia. However, there is no conventional method for efficiently treating an electrocardiogram measured for the long term and types of discriminable arrhythmia are limited. In addition, the use of a remote diagnosis server that can perform a great deal of operations is necessary for accurate reading, but in this case, there is a problem in that a lot of analysis time and computing resources are required.

### SUMMARY

Embodiments of the present disclosure are intended to provide technical means for ensuring accuracy of electrocardiogram analysis while at the same time effectively saving computing resources for electrocardiogram analysis.

According to an aspect of the present disclosure, there is an electrocardiogram analysis apparatus including an inputter configured to receive an electrocardiogram of a subject, a beat analyzer configured to first detect and classify one or more normal beats among a plurality of beats included in the received electrocardiogram, request beat classification for remaining beats except for the one or more normal beats to a remote diagnosis server, and label each of the plurality of beats according to a normal beat detection result and a beat classification result received from the remote diagnosis server, and a rhythm analyzer configured to perform rhythm analysis on the electrocardiogram based on a labeling result.

The electrocardiogram analysis apparatus may further include a validity determiner configured to determine validity of the received electrocardiogram and output a warning message when a determination result does not correspond to a valid electrocardiogram.

The beat analyzer may extract a plurality of first partial signals from the electrocardiogram, detect waveform information of one or more classification target beats included in the extracted first partial signals, and determine whether the one or more classification target beats are the one or more normal beats using one or more of a machine learning classifier that uses the waveform information as an input value, a signal obtained by performing wavelet transformation on the one or more classification target beats, and features of the waveform information.

The machine learning classifier may receive one or more pieces of information among time and magnitude information of a waveform detected from the one or more classification target beats as an input, determine whether the one or more classification target beats are the one or more normal beats and set a threshold value for normal beat classification to 0.9 or more.

The beat analyzer may determine that the one or more classification target beats are the one or more normal beats when a wavelet-transformed signal of the one or more classification target beats satisfies at least one of the following first and second conditions:
first condition: there are two or more extreme values that can be read as T-on or T-off after S-peak and a zero-crossing is present between T-on and T-off; and
second condition: there are two or more extreme values that can be read as P-on or P-off before Q-peak and a zero-crossing is present between P-on and P-off.

The beat analyzer may determine that the one or more classification target beats are the one or more normal beats when a waveform of the one or more classification target beats satisfies at least one of the following third to sixth conditions:
third condition: a magnitude m(R) of R-peak is greater than a greater one (max(m(Q), m(S))) among magnitudes of Q-peak and S-peak (m(R)>max(m(Q), m(S)));
fourth condition: a magnitude m(Pₚₑₐₖ) of P-peak is greater than a greater one (max(m(Pₒₙ), m(P_{off}))) among magnitudes of P-on and P-off (m(Pₚₑₐₖ)>max(m(Pₒₙ), m(P_{off})));
fifth condition: a magnitude m(Tₚₑₐₖ) of T-peak is greater than a greater one (max(m(Tₒₙ), m(T_{off}))) among magnitudes of T-on and T-off (m(Tₚₑₐₖ)>max(m(Tₒₙ), m(T_{off}))); and
sixth condition: a ratio of an interval RR₀ between R-peak and previous R-peak to an interval RR₋₁ between the previous R-peak and R-peak before last of the beat to be analyzed is greater than or equal to 0.9.

The rhythm analyzer may extract a second partial signal including a plurality of beats from the electrocardiogram and compare a classification result of each beat included in the extracted second partial signal and one or more features extracted from the second partial signal with preset rhythm classification rules to classify rhythms included in the second partial signal.

According to another aspect of the present disclosure, there is an electrocardiogram analysis method, which is a method performed in a computing device including one or more processors and a memory for storing one or more programs executed by the one or more processors, including receiving an electrocardiogram of a subject, first detecting one or more normal beats among a plurality of beats included in the received electrocardiogram, requesting beat classification for remaining beats except for the one or more normal beats among the plurality of beats to a remote diagnosis server and receiving a beat classification result from the remote diagnosis server, labeling each of the plurality of beats according to a normal beat detection result and the beat classification result, and performing rhythm analysis on the electrocardiogram based on a labeling result.

The receiving of the electrocardiogram may further include determining validity of the received electrocardiogram and outputting a warning message when a determination result does not correspond to a valid electrocardiogram.

The first detecting of the one or more normal beats may further include extracting a plurality of first partial signals from the electrocardiogram, detecting waveform information of one or more classification target beats included in the extracted first partial signals, and determining whether the one or more classification target beats are the one or more normal beats using one or more of a machine learning classifier that uses the waveform information as an input value, a signal obtained by performing wavelet transformation on the one or more classification target beats, and features of the waveform information.

The machine learning classifier may be configured to receive one or more pieces of information among time and magnitude information of a waveform detected from the one or more classification target beats as an input, determine whether the one or more classification target beats are the one or more normal beats and set a threshold value for normal beat classification to 0.9 or more.

The determining whether or not the one or more classification target beats are the one or more normal beats may be configured to determine that the one or more classification target beats are the one or more normal beats when a wavelet-transformed signal of the one or more classification target beats satisfies at least one of the following first and second conditions:
first condition: there are two or more extreme values that can be read as T-on or T-off after S-peak and a zero-crossing is present between T-on and T-off; and
second condition: there are two or more extreme values that can be read as P-on or P-off before Q-peak and a zero-crossing is present between P-on and P-off.

The determining whether or not the one or more classification target beats are the one or more normal beats may be configured to determine that the one or more classification target beats are the one or more normal beats when a waveform of the one or more classification target beats satisfies at least one of the following third to sixth conditions:
third condition: a magnitude m(R) of R-peak is greater than a greater one (max(m(Q), m(S))) among magnitudes of Q-peak and S-peak (m(R)>max(m(Q), m(S)));
fourth condition: a magnitude m(Pₚₑₐₖ) of P-peak is greater than a greater one (max(m(Pₒₙ), m(P_{off}))) among magnitudes of P-on and P-off (m(Pₚₑₐₖ)>max(m(Pₒₙ), m(P_{off})));
fifth condition: a magnitude m(Tₚₑₐₖ) of T-peak is greater than a greater one (max(m(Tₒₙ), m(T_{off}))) among magnitudes of T-on and T-off (m(Tₚₑₐₖ)>max(m(Tₒₙ), m(T_{off}))); and
sixth condition: a ratio of an interval RR₀ between R-peak and previous R-peak to an interval RR₋₁ between the previous R-peak and R-peak before last of the beat to be analyzed is greater than or equal to 0.9.

The performing of the rhythm analysis may further include extracting a second partial signal including a plurality of beats from the electrocardiogram and comparing a classification result of each beat included in the extracted second partial signal and one or more features extracted from the second partial signal with preset rhythm classification rules to classify rhythms included in the second partial signal.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a block diagram for describing an electrocardiogram analysis system (100) according to an embodiment;
FIG. 2 is a block diagram for describing an electrocardiogram analysis apparatus (102) according to an embodiment;
FIG. 3 is a graph showing examples of a signal of any one beat among an electrocardiogram and a signal which is obtained by performing wavelet transformation on the one beat;
FIG. 4 is a diagram illustrating an example of a process of extracting a second partial signal from an electrocardiogram;
FIG. 5 is a flowchart for describing an electrocardiogram analysis method (500) according to an embodiment; and
FIG. 6 is a block diagram for illustrating and describing a computing environment (10) that includes a computing device appropriate for use in exemplary embodiments.

### DETAILED DESCRIPTION OF EXEMPLARY EMBODIMENTS

Hereinafter, embodiments of the present disclosure will be described in detail with reference to the accompanying drawings. The following detailed description is provided to assist the reader in gaining a comprehensive understanding of the methods, apparatuses, and/or systems described herein. However, the description is only exemplary, and the present disclosure is not limited thereto.

In describing embodiments of the present disclosure, when it is determined that a detailed description of known techniques associated with the present disclosure would unnecessarily obscure the subject matter of the present disclosure, the detailed description thereof will be omitted. Also, terms used herein are defined in consideration of the functions of the present disclosure and may be changed depending on a user, the intent of an operator, or a custom. Accordingly, the terms should be defined based on the following overall description of this specification. The terminology used herein is only for the purpose of describing embodiments of the present disclosure and is not restrictive. The singular forms "a," "an," and "the" are intended to include the plural forms as well, unless the context clearly indicates otherwise. It should be understood that the terms "comprises," "comprising," "includes," and/or "including," specify the presence of stated features, integers, steps, operations, elements, components, and/or groups thereof when used herein, but do not preclude the presence or addition of one or more other features, integers, steps, operations, elements, components, and/or groups thereof.

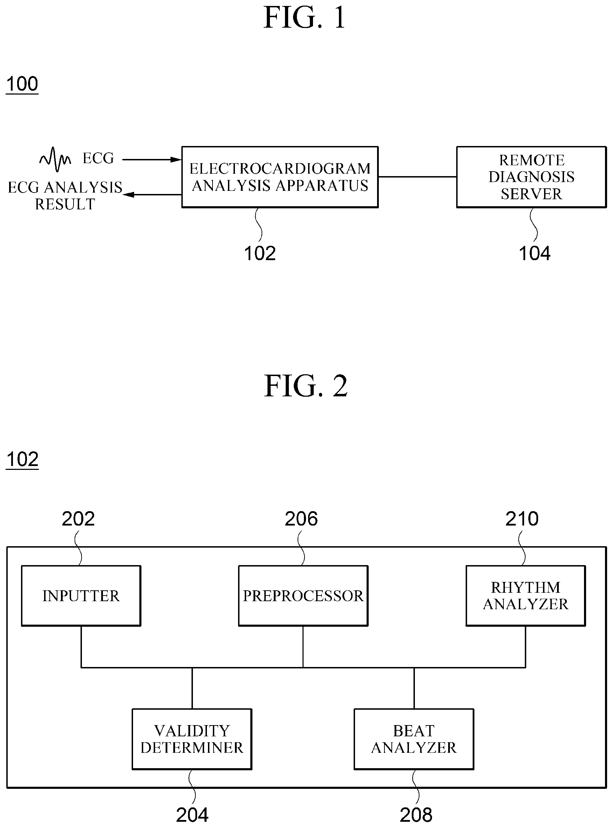

FIG. 1 is a block diagram for describing an electrocardiogram analysis system 100 according to an embodiment. As illustrated in the drawing, the electrocardiogram analysis system 100 according to the embodiment includes an electrocardiogram analysis apparatus 102 and a remote diagnosis server 104.

The electrocardiogram analysis apparatus 102 receives an electrocardiogram of a subject and performs beat analysis and rhythm analysis on the received electrocardiogram. In embodiments of the present disclosure, beat analysis of an electrocardiogram refers to analyzing a feature of each of beats constituting an electrocardiogram to determine a type of each beat. In addition, rhythm analysis of an electrocardiogram refers to analyzing a characteristic of a specific section of an electrocardiogram composed of a plurality of beats to determine a type of a rhythm corresponding to the corresponding section.

In an embodiment, the electrocardiogram analysis apparatus 102 first detects and classifies one or more normal beats among a plurality of beats included in the received electrocardiogram and requests beat classification for remaining beats except for the one or more normal beats from the remote diagnosis server 104. Thereafter, when a result of the beat classification for the remaining beats is received from the remote diagnosis server 104, the electrocardiogram analysis apparatus 102 is configured to label each of the plurality of beats according to the result of the normal beat detection performed directly and the beat classification result received from the remote diagnosis server 104 and to perform rhythm analysis on the electrocardiogram based on a labeling result.

The remote diagnosis server 104 receives a beat classification request from the electrocardiogram analysis apparatus 102, determines a type of the requested beat, and provides the type of the beat to the electrocardiogram analysis apparatus 102. In an embodiment, the remote diagnosis server 104 may determine the type of the beat using a rule-based or machine learning-based beat classification algorithm.

In an embodiment, the electrocardiogram analysis apparatus 102 and the remote diagnosis server 104 may be connected via a communication network. The communication network may include the Internet, one or more local area networks, wire area networks, cellular networks, mobile networks, other types of networks, or combinations of thereof.

FIG. 2 is a block diagram for describing the electrocardiogram analysis apparatus 102 according to the embodiment. As illustrated in the drawing, the electrocardiogram analysis apparatus 102 according to the embodiment includes an inputter 202, a validity determiner 204, a preprocessor 206, a beat analyzer 208, and a rhythm analyzer 210.

The inputter 202 receives an electrocardiogram from a subject to be measured. In an embodiment, the inputter 202 may include its own means for measuring the electrocardiogram, for example, a multi-channel electrocardiogram measuring device or the like, or be connected to a separate electrocardiogram measuring means in a wired or wireless communication manner to obtain the electrocardiogram. In another embodiment, the inputter 202 may be configured to obtain the electrocardiogram by measuring the electrocardiogram using a wearable device worn by the subject or a smart device such as a smart phone possessed by the subject.

The validity determiner 204 determines validity of the electrocardiogram measured by the inputter 202. For example, when the electrocardiogram measuring means is too loosely attached to the subject or when the electrocardiogram measuring device is incorrectly set, such as when the electrocardiogram measuring device is attached at an incorrect position, a meaningless signal may be measured rather than an electrocardiogram signal. When the meaningless signal is input for a long time because the subject or the person in charge of the measurement does not recognize what was done incorrectly, it may lead to unnecessary waste of time and resources. Therefore, the validity determiner 204 determines the validity of the measured electrocardiogram and transmits only the electrocardiogram that is determined to be valid to the preprocessor 206. As a result of the determination, when the measured signal does not correspond to a valid electrocardiogram, the validity determiner 204 deletes the corresponding electrocardiogram. In this case, the validity determiner 204 may output a separate warning message.

In an embodiment, the validity determiner 204 may determine that the measured electrocardiogram is not valid when a change in periodicity of the measured electrocardiogram is detected to be greater than a threshold value, when a signal having a magnitude greater than or equal to a certain range is observed in a time and/or frequency domain, or when a large power greater than a threshold value is detected in a high frequency band (e.g., 30 Hz or more). When it is determined that the measured electrocardiogram is not valid, the validity determiner 204 may output a warning message in real time. In this case, the warning message may include all types of message transmission means capable of providing an audible or visual notification to the subject or the person in charge of measurement, such as an alarm sound, light, a warning popup, or the like.

Relatively complex calculations are not required to determine the validity of the measured electrocardiogram. Therefore, the electrocardiogram analysis apparatus 102 itself may sufficiently determine the validity in real time without the measured electrocardiogram going through the remote diagnosis server 104. That is, according to the embodiments of the present disclosure, by primarily determining the validity of the electrocardiogram in the electrocardiogram analysis apparatus 102, when an invalid electrocardiogram is input, a user may be rapidly alerted about the invalid electrocardiogram so that an abnormality of the measuring device may be rapidly corrected.

The preprocessor 206 performs preprocessing on the electrocardiogram that is determined as being valid by the validity determiner 204. In an embodiment, the preprocessor 206 may remove noise of the measured electrocardiogram using various preprocessing algorithms, such as filtering, smoothing, and the like. The measured electrocardiogram may include not only signals from the heart but also noise caused by body movements, muscle use, changes in measurement baselines, alternating current (AC) interference, and the like. The preprocessor 206 removes such noise from the measured electrocardiogram and provides only the pure electrocardiogram to the beat analyzer 208 and the rhythm analyzer 210.

The beat analyzer 208 analyzes beats constituting the electrocardiogram to classify types of the beats. In an embodiment, the beat analyzer 208 first detects and classifies one or more normal beats among a plurality of beats included in the received electrocardiogram, requests beat classification for remaining beats except for the one or more normal beats to the remote diagnosis server, and labels each of the plurality of beats according to a normal beat detection result and a beat classification result received from the remote diagnosis server.

The beats constituting the electrocardiogram may be divided into various types of beats according to their forms, including the one or more normal beats. Table 1 shows types of beats that are readable in the electrocardiogram.

**[Table 1]**

| Item | Meaning |
|---|---|
| N | Normal beat |
| L | Left bundle branch block beat |
| R | Right bundle branch block beat |
| a | Aberrated atrial premature beat |
| A | Atrial premature beat |
| J | Nodal (junctional) premature beat |
| S | Supraventricular premature or ectopic beat (atrial or nodal) |
| e | Atrial escape beat |
| j | Nodal (junctional) escape beat |
| n | Supraventricular escape beat (atrial or nodal) |
| V | Premature ventricular contraction |
| r | R-on-T premature ventricular contraction |
| E | Ventricular escape beat |
| ! | Ventricular flutter wave |
| F | Fusion of ventricular and normal beat |
| / | Paced beat |
| f | Fusion of paced and normal beat |
| P | Paced beat |
| Q | Unclassifiable beat |

For the beat analysis, the beat analyzer 208 first extracts a partial signal (a first partial signal) of a specific length from the measured electrocardiogram. The first partial signal may be extracted from the electrocardiogram so as to include at least one beat. In an embodiment, the beat analyzer 208 may extract the first partial signal so as to include a plurality of beats, for example, three beats. In another embodiment, the beat analyzer 208 may extract a beat of a specific length, for example, a beat of a length of one second to 1.5 seconds, from the electrocardiogram as the first partial signal. A length of the first partial signal extracted by the beat analyzer 208 or the number of beats may be appropriately determined in consideration of the characteristic of the electrocardiogram or the like. When the extracted first partial signal includes a plurality of beats, the beat analyzer 208 may be configured to perform analysis on one of the beats. Further, the plurality of first partial signals extracted by the beat analyzer 208 may be extracted so that some beats overlap.

When the first partial signal is extracted, the beat analyzer 208 next detects a waveform from the extracted first partial signal. In the case of an electrocardiogram measured from a normal heartbeat, a pattern of a specific waveform having a P-wave, a QRS-complex, and a T-wave is observed due to contraction and relaxation of the heart, and the beat analyzer 208 may use waveform information as a feature for beat analysis.

A peak point and on and/or off points of waveforms included in one beat may be detected as a zero-crossing, an extreme value, etc. of the signal converted by performing wavelet transformation on the first partial signal. The following exemplifies values which are detectable from the beats included in the first partial signal.

R-peak: denotes a maximum value of the electrocardiogram that appears periodically.

Q-peak or S-peak: denotes a minimum value of the electrocardiogram in a 0.1 second section before R-peak or after R-peak

P-on and P-off: denote extreme values (values with a first derivative of 0) of the wavelet-transformed signal before Q-peak as being designated as P-on and P-off, respectively. When there is no extreme value, a point 0.16 seconds before Q-peak is designated as P-on, a point 0.14 seconds before is designated as P-peak, and a point 0.12 seconds before is designated as P-off. When there is one extreme value, the corresponding extreme value is designated as P-on, a point 0.1 seconds after P-on is designated as P-off, and an intermediate value there between is designated as P-peak. When there are two extreme values, one extreme value having a maximum value of the wavelet transformation signal is designated as P-on and the other extreme value is designated as P-off.

P-peak: denotes a zero-crossing of the wavelet transformation signal between P-on and P-off. When there is no zero-crossing, an intermediate value between P-on and P-off is designated as P-peak.

T-on, T-off, and T-peak: denote extreme values of the wavelet transformation signal after S-peak as being designated as T-on and T-off, respectively, and a zero-crossing therebetween as being designated as T-peak (exception: same as P-wave).

T-peak: denotes a zero-crossing of the wavelet transformation signal between T-on and T-off. When there is no zero-crossing, an intermediate value between T-on and T-off is designated as T-peak.

Next, when the waveform is detected as described above, the beat analyzer 208 first determines whether the beats included in the first partial signal are normal beats. For most subjects, normal beats occupy the largest proportion of measured electrocardiograms. Further, the normal beats have features of which shapes are constant and change little compared with other types of beats. Therefore, in the embodiment of the present disclosure, the beat analyzer 208 is configured to detect the one or more normal beat of the typical waveform by itself first and then transmit only the remaining beats to the remote diagnosis server 104 to perform beat analysis. Since a great deal of computing resources are not required to detect one or more typical normal beats first in a simple method, the electrocardiogram analysis apparatus 102 itself may be sufficient to detect the one or more normal beats. Therefore, with the above configuration, there is an effect that can effectively reduce the resource usage of the remote diagnosis server 104.

In an embodiment, the beat analyzer 208 may determine whether the one or more classification target beats are one or more normal beats using one or more of a machine learning classifier that uses the waveform information as an input value, a signal obtained by performing wavelet transformation on the one or more classification target beats, and features of the waveform information. Specifically, the determination of whether or not the one or more classification target beats is the one or more normal beats may be performed using the following three conditions.

### [Condition 1] Normal and abnormal classification using machine learning with inputs of time and magnitude information of a waveform included in each beat

In Condition 1, the beat analyzer 208 may input one or more pieces of time and magnitude information of a waveform included in each beat to a machine learning classifier and determine whether the corresponding beat is normal or abnormal according to a classification result of the corresponding classifier. In this case, the classifier may be a classifier that is lightweight enough to operate in a wearable device or a mobile device.

For example, it is assumed that a normal and/or abnormal classifier using the time information of the waveform included in each beat is g₁, and times of P-on, P-peak, P-off, Q-peak, R-peak, S-peak, T-on, T-peak, and T-off of each beat are t(Pₒₙ), t(Pₚₑₐₖ), t(P_{off}), t(Q), t(R), t(S), t(Tₒₙ), t(Tₚₑₐₖ), and t(T_{off}), respectively. In this case, the electrocardiogram analysis apparatus 102 may determine whether the corresponding beat is normal or abnormal using Equation 1 below. ${g}_{1} \left(\left[t\left({P}_{on}\right),t\left({P}_{peak}\right),t\left({P}_{off}\right),t\left(Q\right),t\left(R\right),t\left(S\right),t\left({T}_{on}\right),t\left({T}_{peak}\right),t\left({T}_{off}\right)\right]\right) = { \begin{matrix}1 , & {if g}_{1} \left(⋅\right) \geq thr . , \\ 0 , & else\end{matrix}$

Specifically, the beat analyzer 208 may determine that the corresponding beat is a normal beat when an output value of the classifier g₁ is greater than or equal to a threshold value. In this case, the threshold value may be set to a very high value, for example, 0.9 or more, and preferably, 0.98 or more. As described above, when the threshold value is set to a very high value, only beats that have features of typical normal beats are classified as normal beats and beats that are slightly out of a normal range are excluded. Therefore, it is possible to prevent the non-normal beats from being classified as the normal beats (false positive).

Next, it is assumed that a normal and/or abnormal classifier using the magnitude information of the waveform included in each beat is g₂, and magnitudes of P-on, P-peak, P-off, Q-peak, R-peak, S-peak, T-on, T-peak, and T-off of each beat are m(Pₒₙ), m(Pₚₑₐₖ), m(P_{off}), m(Q), m(R), m(S), m(Tₒₙ), m(Tₚₑₐₖ), and m(T_{off}), respectively. In this case, the beat analyzer 208 may determine whether the corresponding beat is normal or abnormal using Equation 2 below. ${g}_{1} \left(\left[m\left({P}_{on}\right),m\left({P}_{peak}\right),m\left({P}_{off}\right),m\left(Q\right),m\left(R\right),m\left(S\right),m\left({T}_{on}\right),m\left({T}_{peak}\right),t\left({T}_{off}\right)\right]\right) = { \begin{matrix}1 , & {if g}_{1} \left(⋅\right) \geq thr . , \\ 0 , & else\end{matrix}$

Similar to the case of the classifier g₁, the beat analyzer 208 may determine that the corresponding beat is a normal beat when an output value of the classifier g₂ is greater than or equal to a threshold value. In this case, the threshold value may be set to a very high value, for example, 0.9 or more, and preferably, 0.98 or more. As described above, when the threshold value is set to a very high value, only beats that have features of typical normal beats are classified as normal beats and beats that are slightly out of a normal range are excluded. Therefore, it is possible to prevent the non-normal beat from being classified as the normal beats (false positive). Further, the threshold value of the classifier g₁ and the threshold value of the classifier g₂ may be set to the same value or to different values.

The beat analyzer 208 may determine that Condition 1 is satisfied only when the classification results of the classifiers g₁ and g₂ are all normal.

### [Condition 2] Normal and abnormal classification based on whether a wavelet-transformed signal satisfies a specific condition during a P-Wave and/or T-Wave detection process

In Condition 2, the beat analyzer 208 may determine that Condition 2 is satisfied when the wavelet-transformed signal of the beat to be analyzed satisfies at least one of Conditions 2-1 and 2-2 below.

Condition 2-1: there are two or more extreme values that can be read as T-on and T-off after S-peak and a zero-crossing is present between T-on and T-off.

Condition 2-2: there are two or more extreme values that can be read as P-on and P-off before Q-peak and a zero-crossing is present between P-on and P-off.

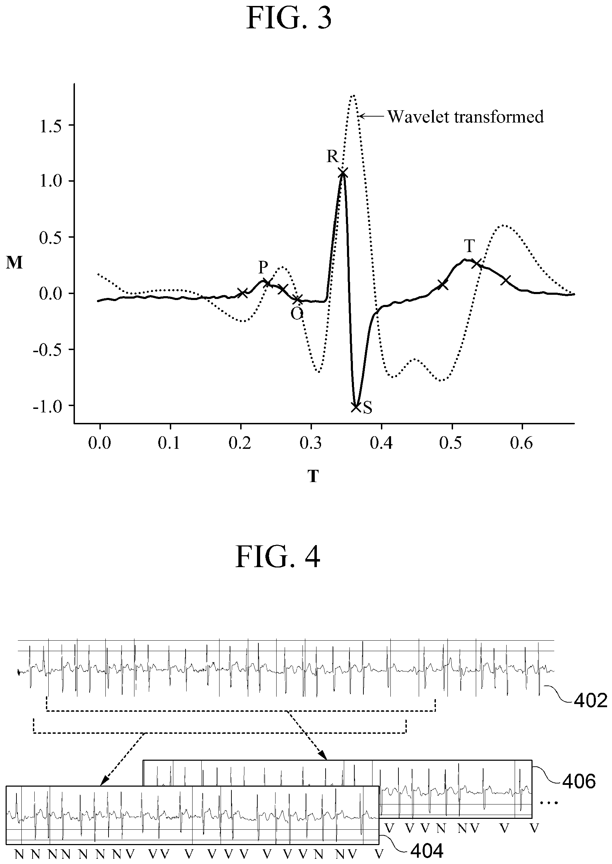

FIG. 3 is a graph showing examples of a signal of any one beat among an electrocardiogram and a signal which is obtained by performing wavelet transformation on the one beat. In the graph shown, a solid line represents any one beat of the electrocardiogram and a dotted line represents a signal obtained by performing wavelet transformation on the one beat. Further, in the graph, a horizontal axis represents time and a vertical axis represents a magnitude of the electrocardiogram. In the graph shown, it can be seen that Condition 2-1 is satisfied because there are two extreme values that can be read as T-on or T-off after S-peak and a zero-crossing is present between T-on and T-off (B). Further, it can be seen that Condition 2-2 is also satisfied because there are two extreme values that can be read as P-on or P-off before Q-peak and a zero-crossing is present between P-on and P-off (A).

### [Condition 3] Normal and abnormal classification based on whether a waveform of a beat satisfies a waveform condition of a normal beat

In Condition 3, the beat analyzer 208 may determine that the corresponding beat satisfies Condition 3 when a waveform of the beat to be analyzed satisfies at least one of Conditions 3-1 to 3-4 below.

Condition 3-1: a magnitude m(R) of R-peak is greater than the greater one (max(m(Q), m(S))) among magnitudes of Q-peak and S-peak (m(R)>max(m(Q), m(S)))
Condition 3-2: a magnitude m(Pₚₑₐₖ) of P-peak is greater than the greater one (max(m(Pₒₙ), m(P_{off}))) among magnitudes of P-on and P-off (m(Pₚₑₐₖ)>max(m(Pₒₙ), m(P_{off})))
Condition 3-3: a magnitude m(Tₚₑₐₖ) of T-peak is greater than the greater one (max(m(Tₒₙ), m(T_{off}))) among magnitudes of T-on and T-off (m(Tₚₑₐₖ)>max(m(Tₒₙ), m(T_{off})))
Condition 3-4: a ratio of an interval RR₀ between R-peak and previous R-peak to an interval RR₋₁ between the previous R-peak and R-peak before last of the beat to be analyzed is greater than or equal to 0.9.

In the embodiments of the present disclosure, the beat analyzer 208 first detects the normal beat in order to reduce the data transmitted to the remote diagnosis server 104 for the beat analysis not by completely classifying normal beats in the entire electrocardiogram but by first filtering some typical normal beats using the electrocardiogram analysis apparatus 102. This is because even when only 50% of the normal beats are first filtered, the time required for the analysis of the entire beats may be reduced by about 30% or more, thereby saving the resources of the remote diagnosis server 104. For this reason, the embodiment of the present disclosure is configured to prevent beats that are not normal beats from being incorrectly classified as normal beats by strictly applying requirements for classifying the normal beats and setting the threshold to a value very close to one.

Next, the beat analyzer 208 transmits the beats not classified as the normal beats to the remote diagnosis server 104 through the above process to request classification for the corresponding beats. That is, in the present embodiment, the beat analyzer 208 is only responsible for classifying typical normal beats, and the remote diagnosis server 104 performs classification by actual beat analysis.

In an embodiment, the remote diagnosis server 104 may classify the beats using a deep neural network. In this case, the remote diagnosis server 104 may extract an additional feature from the beat and use the extracted feature as an input of the classifier or use the extracted feature as it is. The remote diagnosis server 104 may classify types of beats using various methods for classifying beats in addition to the deep neural network. In this case, the types of beat that can be classified are shown in Table 1.

Referring to FIG. 2 again, the rhythm analyzer 210 performs rhythm analysis on the electrocardiogram using the analysis result for each beat of the beat analyzer 208. The rhythms may also be largely classified into normal rhythms and non-normal rhythms, and types of the non-normal rhythms include atrial flutter (AFL), atrial fibrillation (Afib), supraventricular tachyarrhythmia (SVT), ventricular tachycardia (VT) and the like. Specific types of rhythm are shown in Table 2 below.

**[Table 2]**

| Notation | Meaning |
|---|---|
| N | Normal sinus rhythm |
| AFL | Atrial flutter |
| AFIB | Atrial fibrillation |
| Noise | Extreme noise or signal loss in both signals & ECG is unreadable |
| SVTA | Supraventricular tachyarrhythmia |
| VT | Ventricular tachycardia |
| IVR | Idioventricular rhythm |
| VFL. | Ventricular flutter |
| B or VB | Ventricular bigeminy |
| VTr | Ventricular trigeminy |
| SBR | Sinus bradycardia |

In an embodiment, the rhythm analyzer 210 may extract a second partial signal including a plurality of beats from the electrocardiogram and compare a classification result of each beat included in the extracted second partial signal and one or more features extracted from the second partial signal with preset rhythm classification rules to classify rhythms included in the second partial signal. This is described in more detail as follows.

The rhythm analyzer 210 first extracts a partial signal (second partial signal) having a specific length from the electrocardiogram. The second partial signal may be extracted to include a plurality of beats. For example, the rhythm analyzer may be configured to include a certain number of beats (e.g., 21 beats) or to extract a signal of a certain length (e.g., 10 seconds) from the electrocardiogram. In this case, each of the beats included in the second partial signal extracted by the rhythm analyzer 210 is labeled according to the analysis result of the beat analyzer 208. Further, the first partial signal is intended to classify the type of each beat, whereas the second partial signal is intended to grasp the features of consecutive beats, and thus the second partial signal is configured to have a longer length than the first partial signal. The length of the second partial signal extracted by the rhythm analyzer 210 or the number of beats may be appropriately determined in consideration of the characteristic of the electrocardiogram or the like. Further, the plurality of partial signals extracted by the rhythm analyzer 210 may be extracted so that some beats overlap.

FIG. 4 is a diagram illustrating an example of a process of extracting a second partial signal from an electrocardiogram. As illustrated in the drawing, an electrocardiogram 402, which is a target to be analyzed by the rhythm analyzer 210, includes a label for each beat. In the illustrated embodiment, an example in which second partial signals 404 and 406 are extracted by moving a window, which includes 21 beats, beat by beat is illustrated.

Next, the rhythm analyzer 210 classifies rhythms included in the extracted second partial signal. In an embodiment, the rhythm analyzer 210 may compare types of the beats included in the extracted second partial signal and one or more features extracted from the corresponding second partial signal with preset rules to classify the rhythms included in the extracted second partial signal.

Generally, in the case of a normal rhythm, most beats constituting the rhythm are composed of normal beats. However, in the case of an arrhythmia rhythm, non-normal beats increase in weight and show a characteristic pattern. Further, the types and order of the beats configured in the rhythm may be important factors for reading the rhythm. Accordingly, the rhythm analyzer 210 may extract a feature for rhythm reading using the classification result of the beats constituting the rhythm and classify the rhythm using the feature. In an embodiment, the rhythm analyzer 210 may extract a feature using all of the beats included in the read rhythm or may extract a feature in a state in which the read rhythm excludes specific types of beats (V, S, ...) and then may classify the feature. In another embodiment, the rhythm analyzer 210 may extract a feature using only normal beats in the read rhythm and obtain the classification result or may classify the rhythm using information including various types of beat, heart rates, beat intervals, and the like included in one rhythm and features obtained from the information. As described above, according to the embodiments of the present disclosure, the beat reading result for the electrocardiogram signal may be used together with in the rhythm analysis and thus accuracy of the rhythm analysis result may be improved.

Information such as types of beats, heart rates, and beat intervals included in one rhythm and additional features obtained therefrom may be used as the features for rhythm reading. Some of the features that can be used for rhythm reading are listed as follows.

Beat interval change: refers to a difference between intervals RRᵢ and RRᵢ₋₁, which are an R-peak interval of an i^{th} beat and an R-peak interval of the previous beat, among the beats in the rhythm read range. Since a heart rate itself varies by person and situation, the intervals RRi and RRᵢ₋₁ may be used by being normalized by an average beat interval as necessary.

Shannon entropy: entropy of R-peak values of the beats constituting the rhythm may be used. Entropy such as a difference between R-peak and S-peak, a RR interval, or the like may be used as necessary.

Gini score: represents a difference such as R-peak, RS-peak, and the type of the beats of the rhythm to be observed.

Frequency component: the measured signal may be converted into a frequency domain and signal power or phase information in a frequency range of interest (0.5 to 30 Hz) may be used.

The following shows examples of rules that can be used for rhythm reading.
- SVT: read out at a beat interval (RR interval) (120ms<RR int.<600ms).
- SVT start: refers to a point at which the previous RR interval is in the range of 1.2 to 1.35 times longer than the current RR interval.
- SVT end: refers to a point at which the previous RR interval is in the range of 1.3 to 1.35 times longer than current RR interval.
- VT: read at a heart rate (of 160 or more) when a V beat appears three or more times consecutively.
- IVR: read at a heart rate (in a range of 40 to 70) when a V or F beat appears three or more times consecutively and ignored when another beat appears once after three or more consecutive V or F beat appearances.
- VFL: read at a heart rate (160 or more) when a ! beat appears three or more times.
- VB: ignored when an N beat appears twice after three or more consecutive V-N beat appearances in the case in which the N beat appears three or more times consecutively after the V beat appearance (V-N-V-N-V-N-V-N).
- VTr: ignored when another beat appears once after three or more consecutive V-N-N beat appearances in the case in which a V beat appears three or more times consecutively after two N beat appearances (V-N-N-V-N-N-V-N-N).
- Afib or AFL: refers to the case in which the beat interval is irregular. Since classification is difficult under the simple conditions, the classification is performed by inputting a combination of various features into a machine-learned classifier.

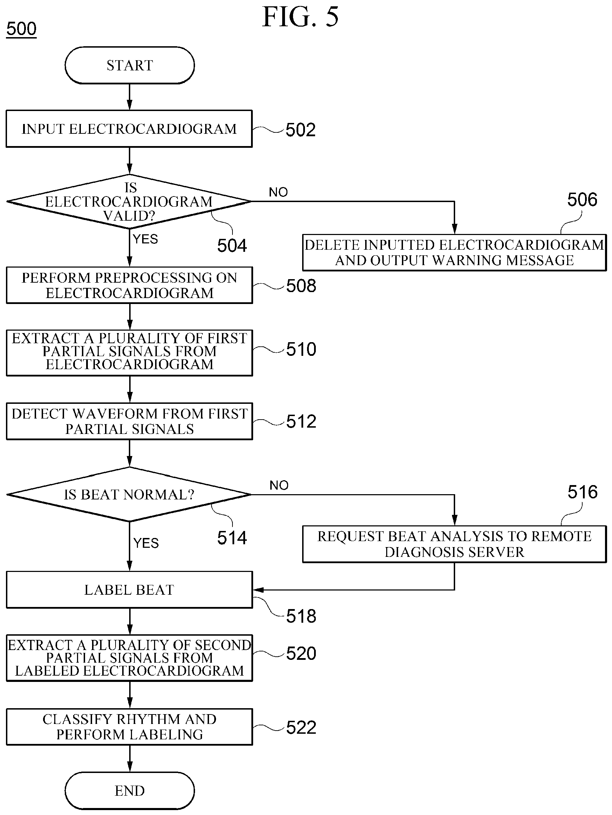

FIG. 5 is a flowchart for describing an electrocardiogram analysis method 500 according to an embodiment. The method illustrated in the flowchart may be performed by a computing device, for example, the electrocardiogram analysis apparatus 102 and the remote diagnosis server 104 described above, including one or more processors and a memory configured to store one or more programs executed by the one or more processors. In the illustrated flowchart, the method or process is described as being divided into a plurality of operations. However, at least some operations may be performed in reverse order, may be performed in combination with another operation, may be omitted, may be performed by being subdivided into sub-operations, or may be performed by adding one or more operations not illustrated.

In operation 502, the inputter 202 of the electrocardiogram analysis apparatus 102 receives an electrocardiogram of a subject.

In operation 504, the validity determiner 204 of the electrocardiogram analysis apparatus 102 determines validity of the received electrocardiogram. As described above, the validity determiner 204 may determine that the measured electrocardiogram is not valid when a change in periodicity of the measured electrocardiogram is detected to be greater than a threshold value, when a signal having a magnitude greater than or equal to a certain range is observed in a time and/or frequency domain, or when a large power greater than a threshold value or more is detected in a high frequency band (e.g., 30 Hz or more).

As a result of the determination in operation 504, when the received electrocardiogram is not valid, the validity determiner 204 may delete the received electrocardiogram and output a warning message in operation 506.

In contrast, as the result of the determination in operation 504, when the received electrocardiogram is valid, the preprocessor 206 performs preprocessing on the received electrocardiogram in operation 508. In an embodiment, the preprocessor 206 may remove noise of the measured electrocardiogram using various preprocessing algorithms, such as filtering, smoothing, and the like.

Thereafter, the beat analyzer 208 of the electrocardiogram analysis apparatus 102 analyzes beats constituting the electrocardiogram to classify types of the beats.

First, in operation 510, the beat analyzer 208 extracts a plurality of first partial signals from the electrocardiogram. In this case, the first partial signals may be extracted from the electrocardiogram to include at least one beat.

In operation 512, the beat analyzer 208 detects waveforms from one or more classification target beats included in the extracted first partial signals. The waveforms detectable from the beats are as described above.

In operation 514, the beat analyzer 208 first determines whether the one or more classification target beats are one or more normal beats based on the waveform information. In an embodiment, the beat analyzer 208 may determine whether the one or more classification target beats are the one or more normal beats using one or more of a machine learning classifier that uses the waveform information as an input value, a signal obtained by performing wavelet transformation on the one or more classification target beats, and features of the waveform information. Since the specific details for determining whether or not the one or more classification target beats are the one or more normal beats in the beat analyzer 208 is described above in detail, repeated descriptions will be omitted.

When a result of the determination in operation 514 does not correspond to the one or more normal beat, the beat analyzer 208 requests beat classification for the remaining beats except for the one or more normal beat to the remote diagnosis server 104 and receives a beat classification result from the remote diagnosis server 104 in operation 516.

In operation 518, the beat analyzer 208 labels each of the plurality of beats included in the electrocardiogram according to a normal beat detection result in operation 514 and a beat classification result received from the remote diagnosis server 104 in operation 516.

After each of the beats is labeled, the rhythm analyzer 210 of the electrocardiogram analysis apparatus 102 performs rhythm analysis on the electrocardiogram using a labeling result for each of the beats of the beat analyzer 208.

Specifically, in operation 520, the rhythm analyzer 210 extracts a second partial signal from the electrocardiogram including the labeling result of each of the beats. In this case, the second partial signal may be extracted to include a plurality of beats.

In operation 522, the rhythm analyzer 210 compares a classification result of the beats included in the extracted second partial signal and one or more features extracted from the second partial signal with preset rhythm classification rules to classify a rhythm included in the second partial signal and labels a rhythm classification result on the electrocardiogram.

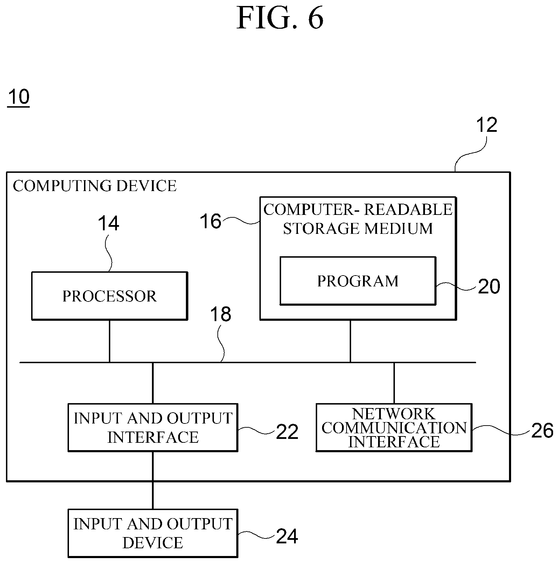

FIG. 6 is a block diagram for illustrating and describing a computing environment 10 that includes a computing device appropriate for use in the exemplary embodiments. In the illustrated embodiment, components may have different functions and capabilities in addition to those described below, and additional components in addition to those described below may be provided.

The illustrated computing environment 10 includes a computing device 12. In an embodiment, the computing device 12 may be an image classification device 100 according to the embodiments of the present disclosure. The computing device 12 includes at least one processor 14, a computer-readable storage medium 16, and a communication bus 18. The processor 14 may allow the computing device 12 to operate according to the exemplary embodiments described above. For example, the processor 14 may execute one or more programs stored in the computer-readable storage medium 16. The one or more programs may include one or more computer executable instructions, and the computer executable instructions may be configured to allow the computing device 12 to perform the operations according to the exemplary embodiments when being executed by the processor 14.

The computer-readable storage medium 16 is configured to store computer executable instructions and program codes, program data, and/or other appropriate forms of information. A program 20 stored in the computer-readable storage medium 16 includes a set of instructions executable by the processor 14. In an embodiment, the computer-readable storage medium 16 may include memories (volatile memories such as random access memories (RAMs), non-volatile memories, or combinations thereof), one or more magnetic disk storage devices, optical disk storage devices, flash memory devices, other types of storage media accessed by the computing device 12 and capable of storing desired information, or appropriate combinations thereof.

The communication bus 18 connects various components of the computing device 12 to each other, including the processor 14 and the computer-readable storage medium 16.

The computing device 12 may further include one or more input and output interfaces 22 for providing an interface for one or more input and output devices 24 and one or more network communication interfaces 26. The input and output interfaces 22 and the network communication interfaces 26 are connected to the communication bus 18. The input and output device 24 may be connected to other components of the computing device 12 through the input and output interfaces 22. For example, the input and output devices 24 may include input devices, such as a pointing device (such as a mouse or trackpad), a keyboard, a touch input device (such as a touchpad or touchscreen), a voice or sound input device, various types of sensor devices, and/or imaging devices, and/or may include output devices, such as display devices, printers, speakers, and/or network cards. For example, the input and output device 24 may be included inside the computing device 12 as one component of the computing device 12 and may be connected to the computing device 12 as a separate device from the computing device 12.

According to the embodiments of the present disclosure, tasks that can be processed in a local device, such as determining the validity of an electrocardiogram measured from a subject and detecting typical normal beats, are performed in the electrocardiogram analysis apparatus first, and only the remaining tasks except for the above tasks are transmitted as a request from the remote diagnosis server, and thus computing resources of the remote diagnosis server and data transmission and reception capacity between the electrocardiogram analysis apparatus and the remote diagnosis server can be saved.

Further, according to the embodiments of the present disclosure, accuracy of rhythm analysis can be improved by utilizing a classification result of each beat obtained as a result of beat analysis of the electrocardiogram in the rhythm analysis.

Embodiments of the present disclosure may include a program for executing the method described herein on a computer and a computer-readable recording medium including the program. The computer-readable recording medium may include any one or a combination of program instructions, a local data file, a local data structure, etc. The medium may be designed and configured specifically for the present disclosure or may be generally available in the field of computer software. Examples of the computer-readable recording medium include a magnetic medium, such as a hard disk, a floppy disk, and a magnetic tape, an optical recording medium, such as a compact disc read only memory (CD-ROM) and a digital video disc (DVD), and a hardware device specially configured to store and execute a program instruction, such as a read only memory (ROM), a RAM, and a flash memory. Examples of the program instructions may include machine code generated by a compiler and high-level language code that can be executed in a computer using an interpreter.

Although example embodiments of the present disclosure have been described in detail, it should be understood by those skilled in the art that various changes may be made without departing from the spirit or scope of the present disclosure. Therefore, the scope of the present disclosure is to be determined by the following claims and their equivalents and is not restricted or limited by the foregoing detailed description.

## Claims

1. An electrocardiogram analysis apparatus comprising:
an inputter configured to receive an electrocardiogram of a subject;
a beat analyzer configured to first detect and classify one or more normal beats among a plurality of beats included in the received electrocardiogram, request beat classification for remaining beats except for the one or more normal beats to a remote diagnosis server, and label each of the plurality of beats according to a normal beat detection result and a beat classification result received from the remote diagnosis server; and
a rhythm analyzer configured to perform rhythm analysis on the electrocardiogram based on a labeling result.

2. The electrocardiogram analysis apparatus of claim 1, further comprising a validity determiner configured to determine validity of the received electrocardiogram and output a warning message when a determination result does not correspond to a valid electrocardiogram.

3. The electrocardiogram analysis apparatus of claim 1, wherein the beat analyzer extracts a plurality of first partial signals from the electrocardiogram, detects waveform information of one or more classification target beats included in the extracted first partial signals, and determines whether the one or more classification target beats are the one or more normal beats using one or more of a machine learning classifier that uses the waveform information as an input value, a signal obtained by performing wavelet transformation on the one or more classification target beats, and features of the waveform information.

4. The electrocardiogram analysis apparatus of claim 3, wherein the machine learning classifier receives one or more pieces of information among time and magnitude information of a waveform detected from the one or more classification target beats as an input, determines whether the one or more classification target beats are the one or more normal beats and sets a threshold value for normal beat classification to 0.9 or more.

5. The electrocardiogram analysis apparatus of claim 3, wherein the beat analyzer determines that the one or more classification target beats are the one or more normal beats when a wavelet-transformed signal of the one or more classification target beats satisfies at least one of a first condition and a second condition below:
first condition: there are two or more extreme values that can be read as T-on or T-off after S-peak and a zero-crossing is present between T-on and T-off; and
second condition: there are two or more extreme values that can be read as P-on or P-off before Q-peak and a zero-crossing is present between P-on and P-off.

6. The electrocardiogram analysis apparatus of claim 3, wherein the beat analyzer determines that the one or more classification target beats are the one or more normal beats when a waveform of the one or more classification target beats satisfies at least one of a third condition, a fourth condition, a fifth condition, and a sixth condition below:
third condition: a magnitude m(R) of R-peak is greater than a greater one (max(m(Q), m(S))) among magnitudes of Q-peak and S-peak (m(R)>max(m(Q), m(S)));
fourth condition: a magnitude m(Pₚₑₐₖ) of P-peak is greater than a greater one (max(m(Pₒₙ), m(P_{off}))) among magnitudes of P-on and P-off (m(Pₚₑₐₖ)>max(m(Pₒₙ), m(P_{off})));
fifth condition: a magnitude m(Tₚₑₐₖ) of T-peak is greater than a greater one (max(m(Tₒₙ), m(T_{off}))) among magnitudes of T-on and T-off (m(Tₚₑₐₖ)>max(m(Tₒₙ), m(T_{off}))); and
sixth condition: a ratio of an interval RR₀ between R-peak and previous R-peak to an interval RR₋₁ between the previous R-peak and R-peak before last of the beat to be analyzed is greater than or equal to 0.9.

7. The electrocardiogram analysis apparatus of claim 1, wherein the rhythm analyzer extracts a second partial signal including a plurality of beats from the electrocardiogram and compares a classification result of each beat included in the extracted second partial signal and one or more features extracted from the second partial signal with preset rhythm classification rules to classify rhythms included in the second partial signal.

8. An electrocardiogram analysis method, which is a method performed in a computing device including one or more processors and a memory for storing one or more programs executed by the one or more processors, the method comprising:
receiving an electrocardiogram of a subject;
first detecting one or more normal beats among a plurality of beats included in the received electrocardiogram;
requesting beat classification for remaining beats except for the one or more normal beats among the plurality of beats to a remote diagnosis server and receiving a beat classification result from the remote diagnosis server;
labeling each of the plurality of beats according to a normal beat detection result and the beat classification result; and
performing rhythm analysis on the electrocardiogram based on a labeling result.

9. The method of claim 8, wherein the receiving of the electrocardiogram further includes:
determining validity of the received electrocardiogram; and
outputting a warning message when a determination result does not correspond to a valid electrocardiogram.

10. The electrocardiogram analysis method of claim 8, wherein the first detecting of the one or more normal beats further includes:
extracting a plurality of first partial signals from the electrocardiogram;
detecting waveform information of one or more classification target beats included in the extracted first partial signals; and
determining whether the one or more classification target beats are the one or more normal beats using one or more of a machine learning classifier that uses the waveform information as an input value, a signal obtained by performing wavelet transformation on the one or more classification target beats, and features of the waveform information.

11. The electrocardiogram analysis method of claim 10, wherein the machine learning classifier is configured to receive one or more pieces of information among time and magnitude information of a waveform detected from the one or more classification target beats as an input, determine whether the one or more classification target beats are the one or more normal beats and set a threshold value for normal beat classification to 0.9 or more.

12. The electrocardiogram analysis method of claim 10, wherein the determining whether or not the one or more classification target beats are the one or more normal beats is configured to determine that the one or more classification target beats are the one or more normal beats when a wavelet-transformed signal of the one or more classification target beats satisfies at least one of a first condition and a second condition below:
first condition: there are two or more extreme values that can be read as T-on or T-off after S-peak and a zero-crossing is present between T-on and T-off; and
second condition: there are two or more extreme values that can be read as P-on or P-off before Q-peak and a zero-crossing is present between P-on and P-off.

13. The electrocardiogram analysis method of claim 10, wherein the determining whether or not the one or more classification target beats are the one or more normal beats is configured to determine that the one or more classification target beats are the one or more normal beats when a waveform of the one or more classification target beats satisfies at least one of a third condition, a fourth condition, a fifth condition, and a sixth condition below:
third condition: a magnitude m(R) of R-peak is greater than a greater one (max(m(Q), m(S))) among magnitudes of Q-peak and S-peak (m(R)>max(m(Q), m(S)));
fourth condition: a magnitude m(Pₚₑₐₖ) of P-peak is greater than a greater one (max(m(Pₒₙ), m(P_{off}))) among magnitudes of P-on and P-off (m(Pₚₑₐₖ)>max(m(Pₒₙ), m(P_{off})));
fifth condition: a magnitude m(Tₚₑₐₖ) of T-peak is greater than a greater one (max(m(Tₒₙ), m(T_{off}))) among magnitudes of T-on and T-off (m(Tₚₑₐₖ)>max(m(Tₒₙ), m(T_{off}))); and
sixth condition: a ratio of an interval RR₀ between R-peak and previous R-peak to an interval RR₋₁ between the previous R-peak and R-peak before last of the beat to be analyzed is greater than or equal to 0.9.

14. The electrocardiogram analysis method of claim 8, wherein the performing of the rhythm analysis further includes:
extracting a second partial signal including a plurality of beats from the electrocardiogram; and
comparing a classification result of each beat included in the extracted second partial signal and one or more features extracted from the second partial signal with preset rhythm classification rules to classify rhythms included in the second partial signal.
